# EUROPÄISCHE PATENTSCHRIFT

(11) **EP 2 274 625 B1**
(45) Veröffentlichungstag und Bekanntmachung des Hinweises auf die Patenterteilung: **29.05.2013**
(21) Anmeldenummer: 09742003.8
(22) Anmeldetag: 28.04.2009
(51) Int. Cl.: G01N 33/86, G01N 33/49

(54) **VORRICHTUNG UND VERFAHREN FÜR DIE BLUTGERINNUNGSDIAGNOSTIK**
APPARATUS AND METHOD FOR BLOOD CLOTTING DIAGNOSTICS
DISPOSITIF ET PROCÉDÉ DE DIAGNOSTIC DE LA COAGULATION DU SANG

(30) Priorität: 08.05.2008 DE 102008022884
(43) Veröffentlichungstag der Anmeldung: 19.01.2011
(73) Patentinhaber: Zander, Rolf, 55124 Mainz (DE)
(72) Erfinder: Zander, Rolf, 55124 Mainz (DE)
(74) Vertreter: WSL Patentanwälte Partnerschaftsgesellschaft
(86) Internationale Anmeldenummer: PCT/EP2009/055145
(87) Internationale Veröffentlichungsnummer: WO 2009/135785

(56) Entgegenhaltungen:
- WO-A2-2008/099179
- US-A- 4 986 964
- US-A1- 2004 214 337
- COLLER B S ET AL: "THE PH DEPENDENCE OF QUANTITATIVE RISTOCETIN-INDUCED PLATELET AGGREGATION: THEORETICAL AND PRACTICAL IMPLICATIONS-A NEW DEVICE FOR MAINTENANCE OF PLATELET-RICH PLASMA PH" BLOOD, AMERICAN SOCIETY OF HEMATOLOGY, US, Bd. 47, Nr. 5, 1. Mai 1976 (1976-05-01), Seiten 841-854, XP008051323 ISSN: 0006-4971
- OUDE EGBRINK M G A ET AL: "Influence of hypercapnia and hypoxia on rabbit platelet aggregation" THROMBOSIS RESEARCH, TARRYTOWN, NY, US, Bd. 57, Nr. 6, 15. März 1990 (1990-03-15), Seiten 863-875, XP022878734 ISSN: 0049-3848 [gefunden am 1990-03-15]

## Beschreibung

Die vorliegende Erfindung betrifft ein Verfahren zur extrakorporalen Bestimmung der Blutgerinnungsaktivität einer Blutprobe eines Individuums, bei dem man die Gerinnungszeit, die vergeht, bis die Blutprobe einen bestimmten Gerinnungsgrad erreicht, oder den Gerinnungsgrad, den die Blutprobe innerhalb einer bestimmten Zeit erreicht, mißt. Darüber hinaus kann man nach diesem Verfahren die extrakorporale Bestimmung der Blutgerinnungsaktivität einer Blutprobe eines Individuums auch dadurch vornehmen, daß man in entsprechender Weise die Fibrinolysezeit, die vergeht, bis eine bis zu einem gewissen Grad geronnene Blutprobe wieder einen bestimmten Fibrinolysegrad erreicht, oder den Fibrinolysegrad, den eine bis zu einem gewissen Grad geronnene Blutprobe innerhalb einer bestimmten Zeit erreicht, mißt. Das Verfahren der vorliegenden Erfindung kann mit einer Vorrichtung zur extrakorporalen Bestimmung der Blutgerinnungsaktivität einer Blutprobe eines Individuums durchgeführt werden, wobei die Vorrichtung eine Meßkammer zur Messung der Gerinnungszeit oder des Gerinnungsgrades bzw. der Fibrinolysezeit oder des Fibrinolysegrades aufweist.

Die vorliegende Erfindung liegt auf dem Gebiet der Gerinnungsdiagnostik. Unter Blutgerinnung ist im Zusammenhang mit dieser Anmeldung das Zusammenspiel der physiologischen Prozesse zu verstehen, die zu einer Gerinnung des Blutes, also zur Ausbildung eines Blutgerinnsels, führen. Die Blutgerinnung (Hämostase) wird im allgemeinen unterteilt in die zelluläre Hämostase und die plasmatische Hämostase. Die zelluläre Hämostase umfaßt die Adhäsion und die Aggregation von Thrombozyten sowie die Aktivierung zusätzlicher Thrombozyten, wobei ein sogenannter "weißer Thrombozytenthrombus" ausgebildet wird. Dagegen bilden während der plasmatischen Hämostase Bestandteile des Blutplasmas ein Maschenwerk aus Fibrinfäden und damit den sogenannten "roten Thrombus", wobei die Rotfärbung durch eingebundene Erythrozyten auftritt. Die plasmatische Hämostase wird ausgelöst und reguliert über die sogenannte Gerinnungskaskade an der eine Reihe verschiedener Gerinnungsfaktoren beteiligt sind. Die vorgenannten Prozesse sind physiologisch und funktionell sehr eng miteinander verbunden und werden hier unter dem Oberbegriff Blutgerinnung zusammengefaßt.

Die Fibrinolyse ist die physiologische Auflösung eines Blutgerinnsels. Hierbei spaltet das Enzym Plasmin die bei der Blutgerinnung entstandenen Fibrinpolymere in verschiedene Abbauprodukte, die über das Blut abtransportiert werden. Dies führt letztlich zur Auflösung eines Blutgerinnsels. Die Fibrinolyse wird bereits mit dem Einsetzen der Blutgerinnung aktiviert und dient, dadurch daß sie der Blutgerinnung antagonistisch entgegen wirkt, der Regulierung der Blutgerinnungsaktivität. Daher umfaßt der Begriff Blutgerinnungsaktivität im Zusammenhang mit der vorliegenden Erfindung nicht nur den Aspekt der Hämostase (Blutgerinnung) sondern auch den der Fibrinolyse (Fibrinspaltung).

Ein Verfahren zur Bestimmung der Blutgerinnungsaktivität ist die Thrombelastographie. Mit diesem Verfahren können sowohl der Aspekt der Blutgerinnung als auch der Aspekt der Fibrinolyse untersucht werden. Weitere Verfahren zur Bestimmung der Blutgerinnungsaktivität sind die Bestimmung der Thromboplastinzeit (TPZ), der aktivierten partiellen Thromboplastinzeit (aPTT), der Thrombinzeit (TT), der Prothrombinzeit, der Ecarin-Gerinnungszeit (ECT) und der aktivierten Gerinnungszeit (ACT) sowie die Impedanz-Aggregometrie oder die Messung der Thrombozyten-Adhäsion und -Aggregation.

Die vorliegende Erfindung zielt insbesondere auf eine Verbesserung der patientennahen Sofort-Diagnostik (*point of care*-Diagnostik, POC) der Blutgerinnung. Die patientennahe Sofort-Diagnostik hat unter anderem den Vorteil, daß die im Rahmen der Diagnostik zu untersuchenden Materialien, beispielsweise dem Körper des zu untersuchenden Individuums entnommene Proben, zeitlich mehr oder weniger unmittelbar nach der Entnahme untersucht werden können. Besonders vorteilhaft ist hierbei, daß bei der Durchführung der Diagnostik auch weitere Aspekte, wie z. B. kurz vor, während oder nach der Entnahme der Probe ermittelte physiologische Parameter des Individuums, berücksichtigt werden können.

Ein menschlicher Patient mit einer Körpertemperatur von 32°C befindet sich im Vergleich zu einem Mensch mit normaler Körpertemperatur in Höhe von etwa 37°C im Zustand der Hypothermie. Im Zustand der Hypothermie ist die Gerinnungsfunktion des menschlichen Blutes erheblich eingeschränkt. Wird für einen hypothermen Patienten eine Gerinnungsdiagnostik vorgenommen, die bei einer über der aktuellen Körpertemperatur dieses Patienten liegenden Temperatur durchgeführt wird, so würde dies zu einer Fehldiagnose führen, da die Blutprobe des hypothermen Patienten, wenn sie während der Diagnostik auf das Niveau der normalen Körpertemperatur zurückgeführt wird (wie bei vielen Geräten üblich), einen normalen Gerinnungsstatus vortäuschen würde, der im tatsächlich kälteren Blut des Patienten gar nicht vorliegt. Einer solchen Fehldiagnose wird daher üblicherweise vorgebeugt, indem man unmittelbar vor, während oder nach der Blutprobenentnahme die Körpertemperatur des Patienten bestimmt und die anschließende Bestimmung der Blutgerinnungsaktivität der aus diesem Patienten entnommenen Blutprobe bei einer auf die bestimmte Körpertemperatur eingestellten Temperatur vornimmt.

US 2004/214337 A1 beschreibt ein Verfahren und eine Vorrichtung für die Blutgerinnungsanalyse, wobei die Messkammer der Messvorrichtung mit einem inerten Gas, wie zum Beispiel Helium, befüllt wird, um jegliche Reaktion der Blutprobe durch deren Kontakt mit Luft zu verhindern. Alternativ wird vorgeschlagen, die Luft aus der Messkammer zu entfernen, um so darin ein Vakuum zu erzeugen.

Coller, B.S. et al. ("The pH dependence of quantitative ristocetin-induced platelet aggregation: Theoretical and practical implications - A new device for maintenance of platelet-rich plasma pH" Blood, American Society of Hematology, US, Bd. 47, Nr. 5, 1. Mai 1976, Seiten 841-854) schlagen für die Blutgerinnungsdiagnostik die Einstellung des pH Wertes auf einen Standard-Wert von 7,7 vor. Hierfür wird eine künstliche Gasatmosphäre in die Messkammer, in der die Blutgerinnungsmessung durchgeführt wird, eingeleitet, um den gewünschten Standard-pH-Wert in der Probe einzustellen und über die Dauer der Gerinnungsmessung konstant zu halten.

Oude Egbrink M.G. A., et al. ("Influence of hypercapnia and hypoxia on rabbit platelet aggregation" Thrombosis Research, Tarrytown, NY, US, Bd. 57, Nr. 6, 15. März 1990. Seiten 863-875) beschreiben Versuche zum Einfluss von Veränderungen des CO₂-Partialdrucks, des pH-Wertes und des O₂-Partialdruckes auf die Aggregation von Blutplättchen von Kaninchen *in vitro.* Hierfür wurde der Blutplättchenaggregationsversuch in Küvetten durchgeführt, die während des Versuches mit einem Gummistopfen abgedeckt wurden, um Verschiebungen bei den Gasdrücken zu verhindern. Anschließend wurden der pH-Wert und die vorgenannten Gaspartialdrücke gemessen.

WO 2008/099179 offenbart einen Apparat, mit dem die Blutgerinnung gemessen werden kann, wobei die Blutprobe hierfür zwischen zwei gegeneinander beweglichen Flächen angeordnet wird, und wobei die Probe im Übrigen von einer inerten Flüssigkeit oder einer kontrollierten Atmosphäre umgeben ist, um die Blutgaszusammensetzung zu kontrollieren.

Wie eingangs bereits erwähnt wurde, bietet die patientennahe Sofort-Diagnostik wesentliche Vorteile gegenüber der Diagnostik zu einem späteren Zeitpunkt, wie z.B. in einem Zentrallabor. Es ist das Ziel der vorliegenden Erfindung, diese Vorteile noch weiter auszubauen, um die in einem Patienten zu einem gegebenen Zeitpunkt vorliegende Blutgerinnungsaktivität noch genauer bestimmen zu können. Es ist daher die Aufgabe der vorliegenden Erfindung, ein entsprechend verbessertes Verfahren zur Verfügung zu stellen, mit dem die Blutgerinnungsaktivität, die das Blut in einem Patienten aufweist, noch genauer analysiert werden kann. Darüber hinaus ist es die Aufgabe der vorliegenden Erfindung, eine Vorrichtung bereitzustellen, mit der ein solches Verfahren durchgeführt werden kann.

Diese Aufgabe wird erfindungsgemäß dadurch gelöst, daß bei dem eingangs genannten Verfahren zur extrakorporalen Bestimmung der Blutgerinnungsaktivität einer Blutprobe eines Individuums die Messung der Gerinnungszeit oder des Gerinnungsgrades bzw. der Fibrinolysezeit oder des Fibrinolysegrades unter Luftabschluß durchgeführt wird und für ein von der Blutprobe abgezweigtes Blutprobenaliquot der pH-Wert bestimmt wird und während der gesamten Messung der Gerinnungs-/Fibrinolysezeit oder des Gerinnungs-/Fibrinolysegrades der vorbestimmte pH-Wert der Blutprobe aufrecht erhalten wird, der für das abgezweigte Blutprobenaliquot bestimmt wurde.

Unter der Gerinnungszeit ist im Zusammenhang mit der vorliegenden Erfindung die Zeit zu verstehen, die vergeht, bis eine Blutprobe einen bestimmten Gerinnungsgrad erreicht. Unter dem Gerinnungsgrad einer Blutprobe ist im Zusammenhang mit der vorliegenden Erfindung das Maß zu verstehen, bis zu dem sich innerhalb eines bestimmten Zeitraumes in der zu untersuchenden Blutprobe ein Blutgerinnsel ausbildet. Unter der Fibrinolysezeit ist im Zusammenhang mit der vorliegenden Erfindung die Zeit zu verstehen, die vergeht, bis sich ein in einer bis zu einem gewissen Grad geronnenen Blutprobe ausgebildetes Blutgerinnsel innerhalb einer bestimmten Zeit bis zu einem gewissen Grad wieder auflöst. Unter dem Fibrinolysegrad einer Blutprobe ist im Zusammenhang mit der vorliegenden Erfindung das Maß zu verstehen, bis zu dem sich ein in einer bis zu einem gewissen Grad geronnenen Blutprobe ausgebildetes Blutgerinnsel innerhalb einer bestimmten Zeit wieder auflöst.

Die Durchführung der Messung unter Luftabschluß bedeutet, daß die Blutprobe wenigstens während der Messung der Blutgerinnungszeit oder des Blutgerinnungsgrades bzw. der Fibrinolysezeit oder des Fibrinolysegrades nicht mit Luft in Kontakt kommt. Anders ausgedrückt bedeutet dies, daß sich die Blutprobe wenigstens während der Messung der vorgenannten Parameter in einer Umgebung befindet, in der keine Luft anwesend ist und in die zumindest während der Meßzeit auch keine Luft eindringen kann.

Unter "Luft" oder "Umgebungsluft" ist hier das Gasgemisch zu verstehen, das den Menschen in seinem alltäglichen Leben umgibt. Demgemäß setzt sich "Luft" oder "Umgebungsluft" im Sinne der vorliegenden Erfindung hauptsächlich aus den Gasen Stickstoff (etwa 78,1 Vol.-%; Partialdruck = 594 mmHg) und Sauerstoff (etwa 20,9 Vol.-%; Partialdruck 159 mmHg) zusammen. Zusätzlich weist die "Luft" oder "Umgebungsluft" noch einen Anteil von etwa 0,9 Vol.-% Argon (Partialdruck 6,8 mmHg) und einen Anteil von etwa 0,03 Vol.-% Kohlenstoffdioxid (Partialdruck 0,2 mmHg) sowie Wasserstoff in Spuren auf. Ferner kann die "natürliche Atmosphäre" einen Anteil an Wasserdampf aufweisen, wobei dieser Anteil im Bereich von 0 bis 6,2 Vol.-% (Partialdruck 0 bis 47 mmHg) liegen und je nach Wasserdampfsättigung und Temperatur stark variieren kann. Der jeweilige Anteil der Gase in der Luft, die nicht Wasserdampf sind, reduziert sich entsprechend des Anteils an Wasserdampf in der Luft. Die Angaben der Partialdrücke der Gase beziehen sich auf einen Barometerdruck von 760 mmHg.

Der Erfindung liegt der Gedanke zugrunde, daß viele physiologische Prozesse pH-abhängig sind, d.h. in einem pH-Optimumsbereich optimal ablaufen und außerhalb dieses Bereiches suboptimal verlaufen oder gar nicht stattfinden können. Es hat sich herausgestellt, daß auch die Blutgerinnung in gewisser Weise vom pH-Wert des Mediums, in dem die Blutgerinnung stattfindet, abhängt. Beispielsweise konnte gezeigt werden, daß manche Blutgerinnungsfaktoren eine vom pH-Wert abhängige Aktivität aufweisen. So kann sich die Aktivität einzelner Gerinnungsfaktoren bei einer Absenkung des pH-Wertes von 7,40 auf 7,20 (entsprechend einem Basendefizit BE von -12,5 mmol/l) um etwa die Hälfte reduzieren und bei einer Erhöhung des pH-Wertes von 7,40 auf 7,60 (entsprechend einem Basenüberschuß BE von +16,5 mmol/l) verdoppeln. Demnach ist es von wesentlicher Bedeutung, daß bei der Bestimmung der Blutgerinnungsaktivität einer Blutprobe der pH-Wert der Blutprobe von der Abnahme beim Patienten bis zum Ende der Messung der Blutgerinnungsaktivität auf einem definierten pH-Niveau gehalten wird. Vorzugsweise entspricht dieses pH-Niveau dem tatsächlich im Blut des Patienten vorliegenden pH-Wert.

Diesem Gedanken trägt die vorliegende Erfindung u.a. dadurch Rechnung, daß die Messung der Blutgerinnungsaktivität unter Luftabschluß durchgeführt wird. Dies hat den Vorteil, daß wenigstens während der Messung kein Gasaustausch zwischen der Blutprobe und der Umgebungsluft stattfinden kann. Die Konzentration bestimmter Gase im Blut hat einen Einfluß auf den pH-Wert des Blutes. So führt der Anstieg der Konzentration von im Blut gelösten CO₂ zu einer Absenkung des pH-Wertes, während die Verringerung der CO₂-Konzentration dazu führt, daß das Blut alkalischer wird.

Handhabt man eine Blut- oder Plasmaprobe so, daß die Probe in Kontakt mit Luft kommen kann, so besteht die Gefahr, daß CO₂ abdiffundieren kann, was zu einer Absenkung der CO₂-Konzentration im Blut führt. Die Absenkung des CO₂-Partialdruckes (pCO₂) von ursprünglich normal 40 mmHg (arterielles Blut) oder ca. 50 mmHg (venöses Blut) auf Werte darunter führt zu einer Alkalisierung der Probe mit einem Anstieg des pH-Wertes auf über den Normalwert von etwa 7,40. Die Messung der Blutgerinnungsaktivität einer Blutprobe, die infolge des Inkontaktkommens mit Luft einen entsprechend erhöhten pH-Wert aufweist, würde daher wegen der zuvor diskutierten pH-Abhängigkeit bestimmter Blutgerinnungsfaktoren zu einem Ergebnis führen, das die tatsächlich im Blut des Patienten vorliegende Blutgerinnungsaktivität nicht exakt wiederspiegelt. Dieser Meßfehler wird erfindungsgemäß dadurch vermieden, daß die Messung der Blutgerinnungsaktivität der Blutprobe unter Luftabschluß durchgeführt wird.

Bei einer bevorzugten Ausführungsform der vorliegenden Erfindung ist das Verfahren zur Bestimmung der Blutgerinnungsaktivität zusätzlich dadurch gekennzeichnet, daß auch die Entnahme der Blutprobe aus dem Körper des Individuums unter Luftabschluß erfolgt. Ein weiteres bevorzugtes Verfahren gemäß der vorliegenden Erfindung ist dadurch gekennzeichnet, daß auch die Aufbewahrung der Blutprobe bis zur Messung unter Luftabschluß erfolgt. Bei einer weiteren bevorzugten Ausführungsform des erfindungsgemäßen Verfahrens erfolgt auch die Überführung der Blutprobe in eine Vorrichtung zur Messung der Blutgerinnungsaktivität unter Luftabschluß. Bei einem insbesondere bevorzugten Verfahren finden sowohl die Entnahme als auch die Aufbewahrung und auch die Überführung der Blutprobe in eine Meßvorrichtung unter Luftabschluß statt. Auf diese Weise wird erreicht, daß die Blutprobe möglichst wenig bis im wesentlichen überhaupt nicht mit Luft in Kontakt kommt, wodurch die Gefahr des Austauschs von Gasen zwischen Blutprobe und Umgebungsluft großteils bis nahezu vollständig ausgeschlossen werden kann, was zu noch exakteren und noch authentischeren Ergebnissen führt.

Bei einer alternativen Ausführungsform der vorliegenden Erfindung erfolgt die Messung der Blutgerinnungsaktivität, ohne daß die Blutprobe während der Messung mit einer Gasphase in Kontakt kommt. Dies kann beispielsweise erreicht werden, indem die Messung der Blutgerinnungsaktivität in einer Meßvorrichtung erfolgt, in der die Blutprobe während der Messung sich in einem Raum befindet, in dem sich weder Luft noch ein anderes Gas befindet. Dies kann beispielsweise dadurch erreicht werden, daß dieser Raum, beispielsweise die in der Meßvorrichtung vorgesehene Meßkammer, vollständig mit der Blutprobe aufgefüllt und anschließend luftdicht abgeschlossen wird. Bei einer bevorzugten Ausführungsform erfolgen auch die Entnahme der Blutprobe und/oder die Aufbewahrung der Blutprobe bis zur Messung und/oder die Überführung der Blutprobe in die Meßvorrichtung, ohne daß die Blutprobe mit einer Gasphase in Kontakt kommt.

Bei einer alternativen Ausführungsform der Erfindung wird die in der Meßkammer angeordnete Blutprobe mit einer Gas-Sperrflüssigkeit überschichtet, so daß die Blutprobe mit oberhalb dieser Gas-Sperrflüssigkeit vorhandener Gasphase nicht in Kontakt kommen kann. Als Gas-Sperrflüssigkeit ist jede Flüssigkeit geeignet, die dadurch gekennzeichnet ist, daß sie im Vergleich zu Blut eine höhere Viskosität und eine geringere Dichte aufweist und sich auf die Blutprobe aufschichten läßt ohne sich mit der Blutprobe zu vermischen. Außerdem zeichnet sich eine Gas-Sperrflüssigkeit gemäß der vorliegenden Erfindung dadurch aus, daß sie im wesentlichen kein CO₂ und/oder kein O₂ und/oder keinen Wasserdampf aufnimmt bzw. hindurch diffundieren läßt. Solche Flüssigkeiten können auf der Basis von Paraffin- oder Silicon-Ölen bzw. Ethylenglycol hergestellt sein.

Ersatzweise können auch Sperr-Gase, die deutlich schwerer (höhere Dichte) als Luft und leichter (geringere Dichte) als Blut sind, eingesetzt werden, um die Blutprobe zu überschichten. Ein Sperrgas, daß für diese Zwecke beispielsweise geeignet ist, ist Schwefelhexafluorid (SF₆).

Dadurch, daß die Gas-Sperrflüssigkeit im wesentlichen keine Diffusion von Gasen zuläßt, kann auch kein Gasaustausch zwischen der Blutprobe und über der Gas-Sperrflüssigkeit vorhandener Gasphase erfolgen, so daß die Messung der Blutgerinnungsaktivität unter Luftabschluß erfolgen kann.

Bei einer weiteren alternativen Ausführungsform wird die Messung der Blutgerinnungsaktivität gemäß der vorliegenden Erfindung unter einer künstlichen Atmosphäre durchgeführt. Unter "künstliche Atmosphäre" ist hier grundsätzlich jedes Gas bzw. Gasgemisch zu verstehen, das in seiner Art und Zusammensetzung von der Umgebungsluft ("natürliche Atmosphäre") abweicht. Die künstliche Atmosphäre ist vorzugsweise ausgewählt unter einem Gas, welches einen von der natürlichen Umgebungsluft abweichenden vorbestimmten CO₂-Partialdruck, O₂-Partialdruck und/oder H₂O-Partialdruck aufweist, oder einem Schutzgas, welches unter Stickstoffgas und/oder einem Edelgas ausgewählt ist.

Unter dem Partialdruck ist im allgemeinen der Druck zu verstehen, der in einem Gasgemisch einem bestimmten in diesem Gasgemisch enthaltenen Gas zugeordnet werden kann. Der Partialdruck entspricht dabei dem Gesamtdruck, den die Komponente beim alleinigen Ausfüllen des gesamten Volumens ausüben würde. Im Sinne der vorliegenden Erfindung wird unter dem Partialdruck eines in einer Flüssigkeit gelösten Gases der Druck verstanden, der dem Partialdruck des entsprechenden Gases in einer Gasphase, die mit der Flüssigkeit in Kontakt steht, entspricht, welcher dazu führen würde, daß das entsprechende Gas an der Grenzfläche von Gas und Flüssigkeit in einem Diffusionsgleichgewicht steht.

Das bedeutet, daß wann immer im Rahmen dieser Anmeldung vom Partialdruck eines Gases in einer Flüssigkeit die Rede ist, die zweite Definition von Partialdruck anzuwenden ist. Dagegen ist wann immer vom Partialdruck eines Gases in einem Gasgemisch die Rede ist, die erste, allgemeine Definition von Partialdruck anzuwenden.

Das Verfahren der vorliegenden Erfindung ist dadurch gekennzeichnet, daß unmittelbar vor, nach oder während der Entnahme der für die Bestimmung der Blutgerinnungsaktivität bestimmten Blutprobe eine Blutvorprobe entnommen wird oder von der Blutprobe ein Blutprobenaliquot abgezweigt wird. Für diese Blutvorprobe bzw. dieses Blutprobenaliquot werden dann einer oder mehrere der folgenden Parameter bestimmt: CO₂-Partialdruck, O₂-Partialdruck und pH-Wert. Diese Vorgehensweise hat den Vorteil, daß man im folgenden im Zusammenhang mit der Bestimmung der Blutgerinnungsaktivität der Blutprobe überprüfen kann, inwieweit die Blutprobe während der Bestimmung der Blutgerinnungsaktivität den gleichen oder einen abweichenden CO₂-Partialdruck, O₂Partialdruck bzw. pH-Wert aufweist. Das heißt, daß auf diese Weise überprüft werden kann, ob die Bestimmung der Blutgerinnungsaktivität unter physiologischen oder pathologischen Bedingungen, d.h. unter den Bedingungen, die bei der Blutprobenentnahme in dem Patienten vorlagen, erfolgt.

Bei einer bevorzugten Ausführungsform der Erfindung, bei der die Messung der Blutgerinnungsaktivität unter einer künstlichen Atmosphäre erfolgt, entspricht der vorbestimmte CO₂-Partialdruck der künstlichen Atmosphäre dem CO₂-Partialdruck der Blutvorprobe bzw. des Blutprobenaliquots. Bei einer weiteren bevorzugten Ausführungsform entspricht der vorbestimmte O₂-Partialdruck der künstlichen Atmosphäre dem O₂-Partialdruck der Blutvorprobe oder des Blutprobenaliquots. Bei einer weiteren bevorzugten Ausführungsform entsprechen sowohl der CO₂-Partialdruck als auch der O₂-Partialdruck der künstlichen Atmosphäre den Partialdrücken der entsprechenden Gase in der Blutvorprobe bzw. dem Blutprobenaliquot. Bei noch einer bevorzugten Ausführungsform der Erfindung entspricht der vorbestimmte H₂O-Partialdruck der künstlichen Atmosphäre dem H₂O-Partialdruck, der in dieser künstlichen Atmosphäre erreicht wird, wenn bei der Temperatur, bei der die Messung der Blutgerinnungsaktivität durchgeführt wird, Wasserdampfsättigung der künstlichen Atmosphäre vorliegt. Hierdurch wird verhindert, daß sich die Blutprobe durch Verdampfen von Wasser aus der Blutprobe aufkonzentriert, wodurch sich der pH-Wert normalerweise absenken würde. Der gewünschte H₂O-Partialdruck der künstlichen Atmosphäre kann beispielsweise dadurch erreicht werden, daß die künstliche Atmosphäre, bevor sie mit der Blutprobe in Kontakt kommt, durch eine mit Wasser gefüllte Fritte geleitet wird, die auf die Temperatur eingestellt ist, bei der auch die Messung der Blutgerinnungsaktivität durchgeführt wird.

Vorzugsweise erfolgt die Messung der Blutgerinnungsaktivität nach dem erfindungsgemäßen Verfahren bei einer bestimmten Temperatur. Vorzugsweise handelt es sich bei dieser Temperatur um die Körpertemperatur, die kurz vor, nach oder während der Entnahme der Blutprobe beim Patienten gemessen wurde.

Bei einer speziellen, standardisierten Ausführungsform des erfindungsgemäßen Verfahrens wird die Messung der Blutgerinnungsaktivität bei 37°C durchgeführt. Bei einer weiteren speziellen, standardisierten Ausführungsform beträgt der vorbestimmte CO₂-Partialdruck der künstlichen Atmosphäre 40 +/- 2,5 mmHg. Bei einer weiteren speziellen, standardisierten Ausführungsform der Erfindung beträgt der vorbestimmte O₂-Partialdruck der künstlichen Atmosphäre 90 +/- 2,5 mmHg. Der vorbestimmte H₂O-Partialdruck der künstlichen Atmosphäre beträgt bei einer speziellen, standardisierten Ausführungsform der Erfindung 47 +/- 2,5 mmHg.

Bei einer bevorzugten Ausführungsform der Erfindung wird als künstliche Atmosphäre das Gasgemisch Carbogen verwendet. Carbogen ist ein Gasgemisch aus 5 Vol.-% CO₂ (Partialdruck 38 mmHg) und 95 Vol.-% O₂ (Partialdruck 722 mmHg). Der Vorteil der Verwendung von Carbogen liegt darin, daß es eine für die erfindungsgemäße Blutgerinnungsdiagnostik gut geeignete Mischung von Gasen aufweist und relativ kostengünstig erhältlich ist.

Vorzugsweise wird der Fluß der künstlichen Atmosphäre in geeigneter Weise eingestellt. Vorzugsweise wird der Fluß so gering wie möglich gehalten. Besonders bevorzugt ist der Gasfluß pro Minute doppelt so groß wie das Volumen, welches die Meßkammer und die Ein- und Auslässe, die mit der Meßkammer verbunden sind, umfassen. Besonders bevorzugt beträgt der Gasfluß 5 ml/Min. Bei einer alternativen Ausführungsform wird, bevor die Blutprobe in die Meßvorrichtung eingebracht worden ist, die Meßvorrichtung gründlich mit der künstlichen Atmosphäre gespült, um sicherzustellen, daß in der Vorrichtung keine Luft vorliegt und um die inneren Oberflächen der Vorrichtung mit der künstlichen Atmosphäre zu äquilibrieren. Vorzugsweise wird beim Spülen einmalig mit wenigstens dem Dreifachen, vorzugsweise mit dem Fünffachen und besonders bevorzugt mit dem Zehnfachen des Innenvolumens der Meßkammer gespült und die Meßkammer und/oder die Ein-/Auslässe anschließend fest verschlossen, so daß von außen keine Umgebungsluft in die Meßkammer eindringen kann. Zweckmäßigerweise wird in entsprechender Weise verfahren, nachdem die Blutprobe in die Meßkammer eingebracht wurde, um eventuell beim Einbringen der Blutprobe eingedrungene Umgebungsluft durch Spülen des nach dem Einbringen der Blutprobe verbleibenden Gasraumes zu entfernen. Vorzugsweise wird auch hier wenigstens das dreifache, vorzugsweise das fünfache und besonders bevorzugt das zehnfache Volumen des verbleibenden Gasraumes eingesetzt.

Bei der vorliegenden Erfindung wird während der gesamten Messung der Blutgerinnungsaktivität (Gerinnungs-/Fibrinolysezeit bzw. Gerinnungs-/Fibrinolyse-grad) ein vorbestimmter pH-Wert der Blutprobe aufrecht erhalten. Bei diesem aufrecht zu erhaltenden pH-Wert handelt es sich um den pH-Wert, der für eine unmittelbar vor, nach oder während der Entnahme der für die Bestimmung der Blutgerinnungsaktivität bestimmten Blutprobe entnommenen Blutvorprobe oder für ein von der Blutprobe abgezweigtes Blutprobenaliquot bestimmt wurde. Sollen Reagenzien verwendet werden, um spezifische Schritte der Gerinnungskaskade vorübergehend zu blockieren oder zu starten (Citrat, Ca²⁺-Ionen), sind diese bezüglich ihres Volumens (Verdünnung der Probe) so gering wie möglich zu dosieren, und bezüglich ihrer Zusammensetzung so zu wählen (ungepuffert), dass ihre Dosierung den ursprünglichen pH-Wert der Probe nicht verändert.

Um den ursprünglichen pH-Wert der Blutprobe aufrecht zu erhalten, sind verschiedene Punkte zu beachten. So wird bei einer Ausführungsform der vorliegenden Erfindung unbedingt vermieden, daß die Probe stark zentrifugiert wird, wodurch in der Blutprobe gelöste Gase aus der Lösung herausgedrängt werden und ausgasen können. Insbesondere beim Ausgasen von CO₂ kommt es zu einer Veränderung, nämlich zu einem Anstieg des pH-Wertes in der Blutprobe, was hier gerade vermieden werden soll. Das gleiche gilt auch im Hinblick auf das Rühren der Blutprobe. Es ist bei einer bevorzugten Ausführungsform der Erfindung daher unbedingt zu vermeiden, die Blutprobe vor oder während der Messung der Blutgerinnungsaktivität so stark zu rühren, daß vermehrt Gase aus der Blutprobe austreten können. Vorzugsweise wird bei dem erfindungsgemäßen Verfahren daher die Blutprobe gar nicht oder nur so langsam zentrifugiert bzw. gerührt, daß kein erhöhtes Ausgasen zu erwarten ist.

Bei einer bevorzugten Ausführungsform der Erfindung werden der Blutprobe vor oder während der Messung der Blutgerinnungsaktivität keine Reagenzien zugesetzt. Bei einer alternativen Ausführungsform werden der Blutprobe so wenig wie möglich Reagenzien zugesetzt. Diese Reagenzien können beispielsweise ausgewählt sein unter einer Citratlösung und einer Ca²⁺-Lösung. Der obligatorische Zusatz von ungepufferter Tri-Natrium-Citratlösung zur Gerinnungshemmung der Blutprobe sowie der spätere Zusatz von ungepufferter Calcium-Lösung zum Starten der Gerinnungskaskade ist so zu wählen, dass die jeweiligen Konzentrationen möglichst hoch und damit die notwendigen Volumina möglichst klein gehalten werden, um eine verdünnungsbedingte pH-Senkung zu verhindern.

Bei einer weiteren bevorzugten Ausführungsform der Erfindung erfolgt die Zugabe von gegebenenfalls erforderlichen Zusätzen pH-neutral, d.h., daß die Stoffe entweder so gewählt werden, daß sie keinen Einfluß auf den pH-Wert der Blutprobe haben (Stoffe, die in wäßrigem Milieu weder sauer noch alkalisch reagieren), oder bei mehreren Stoffen diese so ausgewählt werden, daß sich der Einfluß der einzelnen Stoffe auf den pH-Wert der Blutprobe neutralisiert. Vorzugsweise sind alle zugesetzten Reagenzien ungepuffert, um den ursprünglichen pH-Wert der Probe nicht durch Puffersubstanzen zu beeinflussen.

Bei einer Ausführungsform ist es bevorzugt, daß das Volumen von gegebenenfalls zuzuführenden Reagenzien im Verhältnis zur Blutprobe so klein wie möglich gehalten wird. Werden beispielsweise Blutproben mit Plasmaexpandem verdünnt, so führt dies zu einer erheblichen Senkung des pH-Wertes (Dilutionsazidose). Bereits eine Verdünnung des Blutes *in vitro* von 1:2 (1 Teil Blut + 1 Teil Verdünnungsflüssikgeit) senkt den pH-Wert von 7,40 auf 7,10. Vorzugsweise umfaßt das Volumen der Summe der zugegebenen Reagenzien höchstens 5 % des Volumens der Blutprobe. Noch bevorzugter umfaßt das Volumen der Summe der zugegebenen Reagenzien höchstens 1 % des Volumens der Blutprobe.

Das oben beschriebene erfindungsgemäße Verfahren zur extrakorporalen Bestimmung der Blutgerinnungsaktivität einer Blutprobe eines Individuums kann mit einer Vorrichtung erfolgen, mit der eine extrakorporale Bestimmung der Blutgerinnungsaktivität einer Blutprobe eines Individuums durchgeführt werden kann. Diese Vorrichtung umfaßt eine Meßkammer zur Messung der Gerinnungszeit, die vergeht, bis die Blutprobe einen bestimmten Gerinnungsgrad erreicht, oder zur Messung des Gerinnungsgrades, den die Blutprobe innerhalb einer bestimmten Zeit erreicht. Gleichsam kann mit der Meßkammer auch die Fibrinolysezeit oder der Fibrinolysegrad einer zuvor bis zu einem gewissen Grad geronnenen Blutprobe gemessen werden. Die Meßkammer ist zur Durchführung deserfindungsgemäßen Verfahrens so gestaltet, daß die Messung der Gerinnungs-/Fibrinolysezeit oder des Gerinnungs-/Fibrinolysegrades unter Luftabschluß erfolgt. Dementsprechend ist die Vorrichtung zur Durchführung des oben diskutierten erfindungsgemäßen Verfahrens bzw. einer Ausführungsform dieses Verfahrens geeignet. Die Vorteile der Messung der Blutgerinnungsaktivität unter Luftabschluß wurden zuvor bereits diskutiert.

Bei einer Ausführungsform des erfindungsgemäßen Verfahrens ist die Meßkammer so gestaltet, daß die Messung ohne Kontakt der Blutprobe mit einer Gasphase erfolgt. Vorzugsweise sind gegebenenfalls vorgesehene Zuleitungen, über die eine Blutprobe oder, falls erforderlich, Reagenzien zugegeben werden können, so gestaltet, daß während des Zuführens der Blutprobe bzw. der Reagenzien kein Kontakt der Blutprobe mit einer Gasphase erfolgt bzw. keine Gasphase über diese Zuleitung in die Meßkammer gelangen kann.

Bei einer alternativen Ausführungsform des erfindungsgemäßen Verfahrens ist die Meßkammer so gestaltet, daß die Messung der Blutgerinnungsaktivität unter einer künstlichen Atmosphäre erfolgen kann. Vorzugsweise weist die Meßkammer bei dem erfindungsgemäßen Verfahren für diese Zwecke Mittel zum kontrollierten Einströmen einer Gasphase in die Meßkammer und/oder Mittel zum kontrollierten Ablassen einer Gasphase aus der Meßkammer auf.

Bei einer Ausführungsform des erfindungsgemäßen Verfahrens ist die Vorrichtung dadurch gekennzeichnet, daß sie Mittel zum Bestimmen von Parametern der in der Meßkammer befindlichen Blutprobe aufweist, wobei diese Parameter ausgewählt sind unter Temperatur, pH-Wert, CO₂-Partialdruck und/oder O₂-Partialdruck. Bei einer weiteren Ausführungsform der Erfindung weist die Vorrichtung Mittel auf, über die eine Sättigung der Gasphase mit Wasserdampf erfolgen kann, wobei diese Mittel vorzugsweise die Gasphase bereits vor dem Eintritt in die Meßkammer mit Wasserdampf sättigen. Bei noch einer weiteren Ausführungsform der Erfindung weist die Vorrichtung Mittel zum Einstellen der Temperatur der Gasphase auf. Bei noch einer weiteren Ausführungsform der Erfindung weist die Vorrichtung Mittel zum Einstellen der Temperatur der in der Meßkammer befindlichen Blutprobe auf. Vorzugsweise handelt es sich hierbei um eine temperierbare Meßkammer, wobei die gesamte Meßkammer auf die gewünschte Temperatur gebracht werden kann.

Bei einer Ausführungsform der Erfindung haben die inneren Oberflächen, die mit der Blutprobe in Kontakt kommen, insbesondere die Meßkammer und die gegebenenfalls vorgesehenen Zuleitungen, über die die Blutprobe in die Meßkammer eingebracht werden kann, geringe Gasabsorptionseigenschaften, um zu verhindern, daß eine Äquilibrierung der Gase Kohlendioxid und Sauerstoff der Blutprobe mit einer Oberflächenschicht erfolgt. Andererseits sollen bei bestimmten Ausführungsformen zumindest die inneren Oberflächen der Meßkammer so gestaltet sein, daß sich an diesen Oberflächen Fibrinfäden anheften können.

Bei einer bevorzugten Ausführungsform der Erfindung ist die Meßkammer zylindrisch.

Bei einer bevorzugten Ausführungsform der Erfindung, beispielsweise einer zylindrischen Meßkammer mit einem rotierenden bzw. oszillierenden Stempel, ist die Meßkammer so gestaltet, dass bei einer während der Messung vollständig gefüllten Meßkammer mit einem Volumen zwischen 100 und 500 µl das Verhältnis der Grenzfläche zwischen Blutprobe und der Oberfläche von Meßkammer zum Volumen der Blutprobe möglichst klein ist. Vorzugsweise liegt das Oberflächen-Volumen-Verhältnis bei einem Meßkammer- und damit Probenvolumen von 100 bis 500 µl in dem Bereich von 10:1 cm⁻¹ bis 25:1 cm⁻¹. Ein Oberflächen-Volumen-Verhältnis von 10:1 cm⁻¹ liegt beispielsweise vor, wenn die Oberfläche 5 cm² und das Blutvolumen 0,5 cm³ (500 µl) beträgt, ein solches von 25:1 cm⁻¹ bei einer Oberfläche von 2,5 cm² und einem Blutvolumen von 0,1 cm³ (100 µl).

In den Fällen, in denen in der Meßkammer während der Messung eine Gasphase gegenwärtig ist, ist das Verhältnis der Grenzfläche zwischen Blutprobe und der Gasphase bezogen auf das Volumen der in der Meßkammer befindlichen Blutprobe zusätzlich so zu gestalten, dass das Oberflächen-Volumen-Verhältnis ebenfalls möglichst klein ist. Dies ist der Fall, wenn ein Wert von 1 : 1 cm⁻¹ oder weniger erreicht wird.

Bei einer Ausführungsform der Erfindung weist die Vorrichtung Mittel zum kontrollierten Zuführen von Reagenzien auf. Ein Beispiel für ein Reagens, das über diese Vorrichtung zugeführt werden kann, ist eine Ca²⁺-Lösung.

Das erfindungsgemäße Verfahren ist nicht nur zur Bestimmung der Blutgerinnungsaktivität einer Vollblutprobe geeignet, sondern kann auch zur Bestimmung der Blutgerinnungsaktivität (Gerinnungs-/Fibrinolysezeit bzw. Gerinnungs-/Fibrinolysegrad) eines aus einer Blutprobe gewonnenen Blutderivats (wie z. B. Plasma) angewendet werden.

Das Verfahren gemäß der vorliegenden Erfindung kann grundsätzlich bei jedem Verfahren zur Bestimmung der Blutgerinnungsaktivität angewendet werden. Vorzugsweise wird es zur Bestimmung der Gerinnungszeit verwendet, wobei die Gerinnungszeit vorzugsweise ausgewählt ist unter der Thromboplastinzeit (TPZ), der aktivierten partiellen Thromboplastinzeit (aPTT), der Thrombinzeit (TT), der Prothrombinzeit, der Ecarin-Gerinnungszeit (ECT) und der aktivierten Gerinnungszeit (ACT).

Darüber hinaus kommt das erfindungsgemäße Verfahren bevorzugt bei der Thrombelastographie, bei der Impedanz-Aggregometrie oder bei der Messung der Thrombozyten-Adhäsion und -Aggregation zum Einsatz.

Bei der Thrombelastographie werden viskoelastische Veränderungen des Vollblutes während der Gerinnselbildung bzw. der Gerinnselauflösung gemessen. Hierfür wird die Blutprobe in eine temperierte Küvette eingebracht. Diese Küvette rotiert bzw. oszilliert gegen einen in die Probe gehängten Stempel. Während die Blutprobe allmählich gerinnt, bildet sich zwischen der Innenwand der Küvette und dem Stempel sukzessive ein Fibringerinnsel aus, über das, nachdem eine gewisse Gerinnselstabilität erreicht wurde, die Rotations- bzw. Oszillationsbewegung der Küvette auf den Stempel übertragen wird. Diese Bewegung wird registriert und in Form eines sogenannten Thrombelastogramms aufgezeichnet. Nach diesem Verfahren läßt sich zum einen die Gerinnungszeit, die vergeht, bis die Blutprobe einen bestimmten Gerinnungsgrad erreicht hat, und zum anderen der Gerinnungsgrad, den die Blutprobe innerhalb einer bestimmten Zeit erreicht, bestimmen. Darüber hinaus kann nach diesem Verfahren auch die Fibrinolysezeit, die vergeht, bis sich das zuvor bis zu einem gewissen Grad gebildete Blutgerinnsel bis zu einem gewissen Grad wieder auflöst, oder der Fibrinolysegrad, den die bis zu einem gewissen Grad zuvor geronnene Blutprobe nach einer bestimmten Zeit wieder erreicht, bestimmt werden.

Für Zwecke der ursprünglichen Offenbarung wird darauf hingewiesen, daß sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

Weitere Merkmale und beispielsweise mögliche Merkmalskombinationen sowie die mit diesen Merkmalen bzw. Merkmalskombinationen verbundenen Vorteile werden anhand der folgenden Beispiele erläutert.

### Beispiele:

Die folgenden Beispiele beschreiben exemplarisch eine patientennahe Sofort-Diagnostik der Blutgerinnung bei einem polytraumatisierten Patienten gemäß der vorliegenden Erfindung.

### Beispiel 1:

Einem Patienten, dessen Blutgerinnungsaktivität mittels Thrombelastographie bestimmt werden soll, werden 350 µl Blut abgenommen. Für die Entnahme der Blutprobe wird eine gasdichte Spritze verwendet, die ein möglichst kleines Volumen einer ungepufferten Tri-Natrium-Citratlösung und einen Magnetrührer enthält. Die Blutprobe befindet sich damit unmittelbar nach deren Entnahme unter anaeroben Bedingungen. Anschließend werden Blut und Citrat-Lösung durch wiederholtes Neigen der Spritze vermischt. Danach wird die Probe in die Meßkammer des auf die Patiententemperatur temperierten Gerätes überführt. Die Probe wird dabei unter eine Gas-Sperrflüssigkeit geschichtet. Schließlich wird mit der Stempelrotation begonnen und ein möglichst kleines Volumen Ca²⁺-Lösung zugegeben, wobei mit der vollständigen Zugabe der Lösung eine Zeitschaltuhr ausgelöst wird, mittels der der zeitliche Ablauf der Gerinnselbildung sowie der Fibrinolyse im Rahmen der durchgeführten Thrombelastographie bestimmt wird.

### Beispiel 2:

Einem Patienten, dessen Blutgerinnungsaktivität mittels Bestimmung der Thrombozyten-Aggregation bestimmt werden soll, werden 150 µl Blut abgenommen. Für die Entnahme der Blutprobe unter anaeroben Bedingungen wird eine Glaskapillare, die auf der Innenseite mit Lithium-Heparinat beschichtet ist verwendet. Danach wird die Probe in die Meßkammer des auf die Patiententemperatur temperierten Gerätes überführt, wobei die Meßkammer zuvor mit einem wasserdampfgesättigten Gasgemisch mit einem pCO₂ von 40 mmHg und pO₂ von 100 mmHg gespült wurde. Dieses Gasgemisch streicht auch während der sich anschließenden Bestimmung der Blutgerinnungsaktivität über die obere Grenzfläche der Blutprobe. Sobald die Blutprobe vollständig in die Meßkammer überführt ist, wird eine Zeitschaltuhr ausgelöst, um den zeitlichen Ablauf der Impedanz-Aggregometrie, d.h. der Messung der Thrombozyten-Aggregation, unter Zusatz kleinster Volumina ungepufferter Reagenzien zu bestimmen.

## Patentansprüche

1. Verfahren zur extrakorporalen Bestimmung der Blutgerinnungsaktivität einer Blutprobe eines Individuums, bei dem man die Gerinnungszeit, die vergeht, bis die Blutprobe einen bestimmten Gerinnungsgrad erreicht, oder den Gerinnungsgrad, den die Blutprobe innerhalb einer bestimmten Zeit erreicht, mißt oder bei dem man in entsprechender Weise die Fibrinolysezeit oder den Fibrinolysegrad einer zuvor bis zu einem gewissen Grad geronnenen Blutprobe mißt, **dadurch gekennzeichnet, daß** für ein von der Blutprobe abgezweigtes Blutprobenaliquot der pH-Wert bestimmt wird, die Messung der Gerinnungs-/Fibrinolysezeit oder des Gerinnungs-/Fibrinolysegrades unter Luftabschluß durchgeführt wird und während der gesamten Messung der Gerinnungs-/Fibrinolysezeit oder des Gerinnungs-/Fibrinolysegrades der vorbestimmte pH-Wert der Blutprobe aufrecht erhalten wird, der für das abgezweigte Blutprobenaliquot bestimmt wurde.

2. Verfahren nach Anspruch 1, **dadurch gekennzeichnet, daß** die Zugabe von Zusätzen zu der Blutprobe pH-neutral erfolgt, indem die zugegebenen Stoffe entweder so gewählt werden, daß sie keinen Einfluß auf den pH-Wert der Blutprobe haben, oder bei mehreren Stoffen diese so ausgewählt werden, daß sich der Einfluß der einzelnen Stoffe auf den pH-Wert der Blutprobe neutralisiert.

3. Verfahren nach Anspruch 1 oder 2, **dadurch gekennzeichnet, daß** das Volumen der Summe der zugegebenen Reagenzien höchstens 5 % des Volumens der Blutprobe umfasst, um den ursprünglichen pH-Wert der Probe nicht durch Verdünnung zu beeinflussen.

4. Verfahren nach einem der Ansprüche 1 bis 3, **dadurch gekennzeichnet, daß** alle zugesetzten Reagenzien ungepuffert sind, um den ursprünglichen pH-Wert der Probe nicht durch Puffersubstanzen zu beeinflussen.

5. Verfahren nach einem der Ansprüche 1 bis 4, **dadurch gekennzeichnet, daß** auch die Entnahme der Blutprobe aus dem Körper des Individuums, die Aufbewahrung der Blutprobe bis zur Messung und die Überführung der Blutprobe in eine Vorrichtung zur Messung der Gerinnungs-/Fibrinolysezeit oder des Gerinnungs-/Fibrinolysegrades unter Luftabschluß erfolgen.

6. Verfahren nach einem der Ansprüche 1 bis 5, **dadurch gekennzeichnet, daß** die Entnahme der Blutprobe mit einer gasdichten Spritze oder mit einer Glaskapillare, die auf der Innenseite mit Lithium-Heparinat beschichtet ist, erfolgt.

7. Verfahren nach einem der Ansprüche 1 bis 6, **dadurch gekennzeichnet, daß** die Messung ohne Kontakt der Blutprobe mit einer Gasphase erfolgt oder unter einer künstlichen Atmosphäre erfolgt, wobei als künstliche Atmosphäre z.B. Carbogen verwendet wird.

8. Verfahren nach einem der Ansprüche 1 bis 6, **dadurch gekennzeichnet, daß** ein SperrGas, das deutlich schwerer als Luft und leichter als Blut ist, wie z.B. Schwefelhexafluorid (SF6), eingesetzt wird, um die in der Meßkammer angeordnete Blutprobe zu überschichten, so daß die Blutprobe nicht mit der oberhalb dieses Sperrgases vorhandenen Gasphase in Kontakt kommen kann.

9. Verfahren nach einem der Ansprüche 1 bis 8, **dadurch gekennzeichnet, daß** die Messung der Gerinnungszeit, des Gerinnungsgrades, der Fibrinolysezeit oder des Fibrinolysegrades mit einer Vorrichtung zur extrakorporalen Bestimmung der Blutgerinnungsaktivität einer Blutprobe erfolgt, deren Meßkammer so gestaltet ist, daß die Messung der Gerinnungs-/Fibrinolysezeit oder des Gerinnungs-/Fibrinolysegrades unter Luftabschluß erfolgt, wobei die Vorrichtung Mittel zum Bestimmen des pH-Werts der in der Meßkammer befindlichen Blutprobe aufweist.

10. Verfahren nach Anspruch 9, **dadurch gekennzeichnet, daß** die inneren Oberflächen der Vorrichtung, die mit der Blutprobe in Kontakt kommen, insbesondere die Meßkammer und die gegebenenfalls vorgesehenen Zuleitungen, über die die Blutprobe in die Meßkammer eingebracht werden kann, geringe Gasabsorptionseigenschaften aufweisen.

11. Verfahren nach einem der Ansprüche 9 bis 10, **dadurch gekennzeichnet, daß** die Meßkammer ein Oberflächen-Volumen-Verhältnis der Oberfläche der Meßkammer, die mit der Blutprobe in Kontakt kommt, zu dem Volumen, das eine Blutprobe mit einem Volumen von 100 bis 500 µl in der Meßkammer einnimmt, in dem Bereich von 10:1 cm⁻¹ bis 25:1 cm⁻¹ aufweist.

12. Verfahren nach einem der Ansprüche 9 bis 11, **dadurch gekennzeichnet, daß** in den Fällen, in denen in der Meßkammer während der Messung eine Gasphase gegenwärtig ist, das Verhältnis der Grenzfläche zwischen Blutprobe und der Gasphase bezogen auf das Volumen der in der Meßkammer befindlichen Blutprobe so zu gestalten ist, dass das Oberflächen-Volumen-Verhältnis höchstens einen Wert von 1 : 1 cm⁻¹ erreicht.

13. Verfahren nach einem der Ansprüche 9 bis 12, **dadurch gekennzeichnet, daß** die Meßkammer so gestaltet ist, dass bei einer während der Messung vollständig gefüllten Meßkammer mit einem Volumen zwischen 100 und 500 µl das Verhältnis der Grenzfläche zwischen Blutprobe und der Oberfläche der Meßkammer zum Volumen der Blutprobe in dem Bereich von 10:1 cm⁻¹ bis 25:1 cm⁻¹ liegt.

14. Verfahren nach einem der vorangehenden Ansprüche, **dadurch gekennzeichnet, daß** die zu bestimmende Gerinnungszeit vorzugsweise ausgewählt ist unter der Thromboplastinzeit (TPZ), der aktivierten partiellen Thromboplastinzeit (aPTT), der Thrombinzeit (TT), der Prothrombinzeit (PT), der Ecarin-Gerinnungszeit (ECT) und der aktivierten Gerinnungszeit (ACT), oder daß das Verfahren bei der Thrombelastographie (TEG), bei der Impedanz-Aggregometrie oder bei der Messung der Thromobozyten-Adhäsion und -Aggregation angewendet wird.

15. Verfahren nach einem der vorangehenden Ansprüche, **dadurch gekennzeichnet, daß** man im Zusammenhang mit der Bestimmung der Blutgerinnungsaktivität der Blutprobe überprüft, inwieweit die Blutprobe während der Bestimmung der Blutgerinnungsaktivität den gleichen pH-Wert aufweist wie bei der Blutprobenentnahme oder einen abweichenden pH-Wert.

## Claims

1. A method of extracorporeal determination of the blood clotting activity of a blood sample of an individual, in which the clotting time which elapses until the blood sample reaches a given degree of clotting or the degree of clotting reached by the blood sample within a given time is measured or in which in a corresponding manner the fibrinolysis time or the degree of fibrinolysis of a blood sample which has previously clotted to a certain degree is measured, **characterised in that** the pH-value is determined for a blood sample aliquot derived from the blood sample, measurement of the clotting/fibrinolysis time or the degree of clotting/fibrinolysis is effected with the exclusion of air and during the entire measurement of the clotting/fibrinolysis time or the degree of clotting/fibrinolysis the predetermined pH-value of the blood sample is maintained, which was determined for the derived blood sample aliquot.

2. A method according to claim 1 **characterised in that** the addition of additives to the blood sample is effected pH-neutral by the added substances either being so selected that they have no influence on the pH-value of the blood sample or in the case of a plurality of substances they are so selected that the influence of the individual substances on the pH-value of the blood sample is neutralised.

3. A method according to claim 1 or claim 2 **characterised in that** the volume of the total of the added reagents includes at most 5% of the volume of the blood sample in order not to influence the original pH-value of the sample by dilution.

4. A method according to one of claims 1 to 3 **characterised in that** all added reagents are unbuffered in order not to influence the original pH-value of the sample by buffer substances.

5. A method according to one of claims 1 to 4 **characterised in that** extraction of the blood sample from the body of the individual, storage of the blood sample until the measurement operation and transfer of the blood sample into an apparatus for measuring the clotting/fibrinolysis time and/or the degree of clotting/fibrinolysis are effected with the exclusion of air.

6. A method according to one of claims 1 to 5 **characterised in that** the blood sample is taken with a gas-tight syringe or with a glass capillary which is coated on the inside with lithium heparinate.

7. A method according to one of claims 1 to 6 **characterised in that** measurement is effected without contact of the blood sample with a gas phase or in an artificial atmosphere, wherein for example carbogen is used as the artificial atmosphere.

8. A method according to one of claims 1 to 6 **characterised in that** a barrier gas which is markedly heavier than air and lighter than blood such as for example sulphur hexafluoride (SF₆) is used to overlay the blood sample arranged in the measurement chamber so that the blood sample cannot come into contact with the gas phase above the barrier gas.

9. A method according to one of claims 1 to 8 **characterised in that** measurement of the clotting time, the fibrinolysis time or the degree of fibrinolysis is effected with an apparatus for the extracorporeal determination of the blood clotting activity of a blood sample, whose measurement chamber is of such a configuration that measurement of the clotting/fibrinolysis time or the degree of clotting/fibrinolysis is effected with the exclusion of air, wherein the apparatus has means for determining the pH-value of the blood sample in the measurement chamber.

10. A method according to claim 9 **characterised in that** the internal surfaces of the apparatus that come into contact with the blood sample, in particular the measurement chamber and the feed lines which are possibly provided and by way of which the blood sample can be introduced into the measurement chamber have low gas absorption properties.

11. A method according to one of claims 9 to 10 **characterised in that** the measurement chamber has a surface-volume ratio of the surface of the measurement chamber, that comes into contact with the blood sample, to the volume that a blood sample of a volume of 100 to 500 µl occupies in the measurement chamber in the range of 10:1 cm⁻¹ to 25:1 cm⁻¹.

12. A method according to one of claims 9 to 11 **characterised in that** in the cases in which a gas phase is present in the measurement chamber during the measurement operation the ratio of the interface between the blood sample and gas phase with respect to the volume of the blood sample in the measurement chamber is to be such that the surface-volume ratio achieves at most a value of 1:1 cm⁻¹.

13. A method according to one of claims 9 to 12 **characterised in that** the measurement chamber is of such a configuration that in the case of a measurement chamber which is completely filled during the measurement operation, of a volume of between 100 and 500 µl, the ratio of the interface between the blood sample and the surface of the measurement chamber to the volume of the blood sample is in the range of 10: 1 cm⁻¹ to 25: 1 cm⁻¹.

14. A method according to one of the preceding claims **characterised in that** the clotting time to be determined is preferably selected from the thromboplastin time (TPT), the activated partial thromboplastin time (aPTT), the thrombin time (TT), the prothrombin time, the ecarin clotting time (ECT) and the activated clotting time (ACT) or that the method is used in thrombelastography (TEG), in impedance aggregometry or in the measurement of thrombocytes adhesion and aggregation.

15. A method according to one of the preceding claims **characterised in that** in connection with determining the blood clotting activity of the blood sample a check is made on the extent to which the blood sample during the operation of determining the blood clotting activity is of the same pH-values as when taking the blood sample or is of a different pH-value.

## Revendications

1. Procédé de détermination extracorporelle de l'activité de coagulation du sang d'un échantillon de sang d'un individu, dans lequel on mesure le temps de coagulation qui s'écoule jusqu'au moment où l'échantillon de sang atteint un degré de coagulation donné, ou le degré de coagulation que l'échantillon de sang atteint dans un temps donné, ou dans lequel on mesure d'une façon appropriée le temps de fibrinolyse ou le degré de fibrinolyse d'un échantillon de sang qui a coagulé préalablement jusqu'à un certain degré, **caractérisé en ce qu'**on détermine le pH pour une partie aliquote de l'échantillon de sang qui a été isolée de l'échantillon de sang, la mesure du temps de coagulation/fibrinolyse ou du degré de coagulation/fibrinolyse est exécutée sous exclusion d'air et, pendant toute la durée de la mesure du temps de coagulation/fibrinolyse ou du degré de coagulation/fibrinolyse, on maintient le pH prédéterminé de l'échantillon de sang qui a été déterminé pour la partie aliquote de l'échantillon de sang qui a été isolée.

2. Procédé selon la revendication 1, **caractérisé en ce que** l'adjonction d'additifs à l'échantillon de sang s'effectue à un pH neutre, du fait que, soit les substances ajoutées sont choisies de manière à n'avoir aucune influence sur le pH de l'échantillon de sang, soit, dans le cas de substances multiples, ces substances sont choisies de manière que les influences des substances individuelles sur le pH de l'échantillon de sang se neutralisent.

3. Procédé selon la revendication 1 ou 2, **caractérisé en ce que** le volume de la somme des réactifs ajoutés représente au maximum 5 % du volume de l'échantillon de sang, afin de ne pas influencer le pH initial de l'échantillon par dilution.

4. Procédé selon l'une des revendications 1 à 3, **caractérisé en ce que** tous les réactifs ajoutés sont non tamponnés, afin de ne pas influencer le pH initial de l'échantillon par des substances tampon.

5. Procédé selon l'une des revendications 1 à 4, **caractérisé en ce que** le prélèvement de l'échantillon de sang sur le corps de l'individu, la conservation de l'échantillon de sang jusqu'à la mesure et le transfert de l'échantillon de sang dans un dispositif pour la mesure du temps de coagulation/fibrinolyse ou du degré de coagulation/fibrinolyse s'effectuent également sous exclusion d'air.

6. Procédé selon l'une des revendications 1 à 5, **caractérisé en ce que** le prélèvement de l'échantillon de sang s'effectue à l'aide d'une seringue étanche aux gaz ou à l'aide d'un capillaire en verre revêtu d'héparinate de lithium sur sa face interne.

7. Procédé selon l'une des revendications 1 à 6, **caractérisé en ce que** la mesure s'effectue sans contact entre l'échantillon de sang et une phase gazeuse, ou sous atmosphère artificielle, auquel cas on utilise par exemple du carbogène comme atmosphère artificielle.

8. Procédé selon l'une des revendications 1 à 6, **caractérisé en ce qu'**on utilise un gaz d'arrêt, qui est notablement plus lourd que l'air et plus léger que le sang, comme, par exemple l'hexafluorure de soufre (SF6) pour recouvrir l'échantillon de sang placé dans la chambre de mesure, de telle manière que l'échantillon de sang ne puisse pas entrer en contact avec la phase gazeuse présente au-dessus de ce gaz d'arrêt.

9. Procédé selon l'une des revendications 1 à 8, **caractérisé en ce que** la mesure du temps de coagulation, du degré de coagulation, du temps de fibrinolyse ou du degré de fibrinolyse s'effectue à l'aide d'un dispositif pour la détermination extracorporelle de l'activité de coagulation d'un échantillon de sang dont la chambre de mesure est constituée de manière que la mesure du temps de coagulation/fibrinolyse ou du degré de coagulation/fibrinolyse s'effectue sous exclusion d'air, l'appareillage comportant des moyens pour la détermination du pH de l'échantillon de sang qui se trouve dans la chambre de mesure.

10. Procédé selon la revendication 9, **caractérisé en ce que** les surfaces internes du dispositif qui entrent en contact avec l'échantillon de sang, en particulier la chambre de mesure et les conduites éventuellement prévues par lesquelles l'échantillon de sang peut être introduit dans la chambre de mesure, présentent de faibles propriétés d'absorption des gaz.

11. Procédé selon la revendication 9 ou 10, **caractérisé en ce que** la chambre de mesure présente un rapport surface-volume liant la surface de la chambre de mesure qui entre en contact avec l'échantillon de sang, au volume qu'un échantillon de sang possédant un volume de 100 à 500 µl occupe dans la chambre de mesure, compris dans la plage allant de 10:1 cm⁻¹ à 25:1 cm⁻¹.

12. Procédé selon l'une des revendications 9 à 11, **caractérisé en ce que**, dans les cas où une phase gazeuse est présente dans la chambre de mesure pendant la mesure, le rapport liant l'interface entre l'échantillon de sang et la phase gazeuse au volume de l'échantillon de sang qui se trouve dans la chambre de mesure doit être établi de manière que le rapport surface-volume atteigne au maximum une valeur de 1:1 cm⁻¹.

13. Procédé selon l'une des revendications 9 à 12, **caractérisé en ce que** la chambre de mesure est conformée de telle manière que, dans le cas où la chambre de mesure possédant un volume compris entre 100 et 500 µl est entièrement remplie pendant la mesure, le rapport liant l'interface entre l'échantillon de sang et la surface de la chambre de mesure au volume de l'échantillon de sang est compris dans la plage allant de 10:1 cm⁻¹ à 25:1 cm⁻¹.

14. Procédé selon l'une des revendications précédentes, **caractérisé en ce que** le temps de coagulation à déterminer est de préférence choisi parmi le temps de thromboplastine (TPZ), le temps de thromboplastine partielle activée (aPTT), le temps de thrombine (TT), le temps de prothrombine (PT), le temps de coagulation par l'écarine (ECT) et le temps de coagulation activée (ACT), ou **en ce que** le procédé est utilisé dans la thrombélastographie (TEG), dans l'agrégométrie par impédance, ou dans la mesure de l'adhésion et de l'agrégation plaquettaires.

15. Procédé selon l'une des revendications précédentes, **caractérisé en ce que**, en corrélation avec la détermination de l'activité de coagulation de l'échantillon de sang, on vérifie dans quelle mesure l'échantillon de sang présente le même pH pendant la détermination de l'activité de coagulation du sang que lors du prélèvement de l'échantillon de sang, ou s'il présente un pH différent.
